# EUROPEAN PATENT APPLICATION

(11) **EP 3 995 812 A1**
(43) Date of publication of application: **11.05.2022**
(21) Application number: 20206687.4
(22) Date of filing: 10.11.2020
(51) Int. Cl.: G01N 21/33, G01N 21/65, G01N 33/18, G01J 3/44, G01N 21/64

(54) **ANALYSIS SYSTEM FOR AQUEOUS TEST LIQUIDS**

(71) Applicant: Artha AG, 8952 Schlieren (CH)
(72) Inventor: PFIFFNER, Peter, 8704 Herrliberg (CH); DENZLER, Daniel, 6030 Ebikon (CH)
(74) Representative: Zimmermann & Partner Patentanwälte mbB

(57) **Abstract**

Embodiments of an analysis system for analysing of components in an aqueous test liquid and a method for analysing of components in an aqueous test liquid are provided herein. The analysis system comprises a deep UV spectroscopy system having a first measurement set-up for measuring a raw Raman spectrum of the test liquid being held in a sample chamber. The raw Raman spectrum comprises the wavelength of a Raman peak of water. The analysis further comprises a processing system for processing the raw Raman spectrum. The processing system is configured to extract the amplitude of the Raman peak of water from the raw Raman spectrum; determine, from the extracted Raman peak of water, a scaling factor; and scale the raw Raman spectrum by the scaling factor. According to an embodiment, the analysis system is further adapted for measuring a raw photoluminescence spectrum, and scaling the raw photoluminescence spectrum by a second scaling factor determined from a second peak of water.

## Description

### FIELD OF THE INVENTION

The present disclosure relates to an analysis system for analysing of components in an aqueous test liquid. Further embodiments relate to a method for analysing of components in an aqueous test liquid.

### BACKGROUND OF THE INVENTION

State-of-the-art methods for the quality control of test liquids include classical chemical analysis techniques. Classical chemical analysis techniques typically comprise the steps of sampling, enrichment (in case of low concentrations), separation of individual components (e.g. by gas chromatography) and detection of the individual components. The last step may be considered as an online or real-time measurement if an online detector is employed, but usually the detection step is not sensitive enough and not selective enough without carrying out the other steps. The limited selectivity usually results from signals of different contaminants overlapping and thus encumbering the interpretation of the measurement. Online detectors are commonly based on the measurement of physical and/or chemical properties, e.g. heat conductivity (e.g. flow controllers), or heat of combustion (e.g. in many hydrogen sensors).

There are a number of systems for the analysis of liquid samples, wherein the detection step can be carried out directly, i.e. without the need for sampling, enrichment and separation of individual components. However, these methods suffer from various drawbacks. For example, Infrared spectroscopy is a promising technique in the gas phase, but suffers from a relatively low sensitivity for aqueous samples, and is non-sensitive for specific compounds, for example soot or conductive samples.

Sewage water analysis is an application for which it is apparent that current state-of-the-art methods for qualitative and quantitative analysis of test samples are non-satisfactory. Sewage water treatment plants have to use elaborated cleaning stages in addition to mechanical and biological treatments to remove environmentally problematic contaminants.

Typical processes are based on initial treatment stages for removing larger contaminants (macro pollutants) as well as partially removing micro pollutants such as sand filtration, and are further based on micropollutant treatment stages such as membrane processes, osmosis, ozonolysis or active carbon filters. Sewage water treatment plants require monitoring and managing the performance of all cleaning stages (such as the exemplary aforementioned cleaning stages). Currently, the only available real-time measurement is UV-absorbance spectroscopy, which has very limited chemical selectivity. Chemically selective information can currently only be obtained from ex-situ, lab-based measurements of (sewage) water samples. A fast feedback to react to spontaneously occurring pollutants is thus impossible.

### BRIEF SUMMARY OF THE INVENTION

In view of the above, there is a need for a system and a method to perform analysis of test liquids, wherein the system is chemically selective and separation of individual components of the test sample prior to analysis of the test sample is not required to obtain information on the concentrations of components or contaminants contained in the aqueous test liquid. Further, there is a need for a system, which can differentiate between possible contributions of individual components of the test sample and/or which is sensitive and therefore especially suitable for detecting low concentrated components/contaminants.

Briefly, an analysis system and a method for analysing of components in an aqueous are provided to overcome at least some of the abovementioned limitations. This object is accomplished by means of an analysis system according to claim 1 and a method according to claim 14.

According to an embodiment of the present disclosure, an analysis system for analysing of components in an aqueous test liquid is provided. The analysis system includes a deep UV spectroscopy system having a first measurement set-up for measuring a raw Raman spectrum of the test liquid being held in a sample chamber. The raw Raman spectrum comprises the wavelength of a Raman peak of water. The analysis system includes a processing system for processing the raw Raman spectrum. The processing system is configured to extract the amplitude of the Raman peak of water from the raw Raman spectrum; determine, from the extracted Raman peak of water, a scaling factor; and scale the raw Raman spectrum by the scaling factor.

According to an embodiment of the present disclosure, a method for analysing of components in an aqueous test liquid is provided. The method includes an analysis system having a deep UV spectroscopy system including a first measurement set-up for measuring a raw Raman spectrum of the test liquid being held in a sample chamber. The raw Raman spectrum comprises the wavelength of a Raman peak of water. The method includes processing the raw Raman spectrum, including extracting the amplitude of the Raman peak of water from the raw Raman spectrum; determining, from the extracted Raman peak of water, a scaling factor; and scaling the raw Raman spectrum by the scaling factor.

Preferably, the aqueous test liquid contains predominantly water (e.g., at least 90% water). While spectroscopic methods allow identifying the presence of substances (contaminants), it is difficult to obtain quantitative information about absolute contents of these substances. For this purpose, it has been usually necessary to perform cumbersome calibration steps prior to each measurement or at least in regular intervals. According to an aspect of the present invention, however, this drawback can be avoided by utilising the knowledge about the water content of the liquid, and by scaling the raw Raman spectrum by the scaling factor extracted from the Raman peak of water associated with the water content. Thereby a scaled Raman spectrum may be obtained that is calibrated due to the known information about the water content of the aqueous liquid, and that therefore allows quantitative information about absolute contents also of other substances without the necessity of further calibration. Alternatively or additionally, the amplitude of the Raman peak of water and/or the scaling factor may be used for cross-checking other calibrations and/or for obtaining diagnostic information about the spectroscopy system.

### BRIEF DESCRIPTION OF THE DRAWINGS

The parts shown in the Figures are not necessarily to scale, instead emphasis being placed upon illustrating the principles of the invention. Moreover, in the Figures, like reference signs designate corresponding parts. In the drawings:
Figure 1 schematically illustrates an analysis system according to an embodiment of the present disclosure.
Figure 2 displays an example of a spectrum recorded with the first measurement set-up of the analysis system according to an embodiment of the present disclosure.
Figure 3 displays an example of a spectrum recorded with the second measurement set-up of the analysis system according to an embodiment of the present disclosure.

### DETAILED DESCRIPTION OF THE INVENTION

In the following detailed description, reference is made to the accompanying drawings, which form a part hereof, and in which are shown by way of illustration specific embodiments in which the invention may be practiced.

As used herein, the terms "having", "containing", "including", "comprising" and the like are open ended terms that indicate the presence of stated elements or features, but do not preclude additional elements or features.

It is to be understood that other embodiments may be utilised and structural or logical changes may be made without departing from the scope of the present invention. The following detailed description, therefore, is not to be taken in a limiting sense, and the scope of the present invention is defined by the appended claims. The embodiments described herein use specific language, which should not be construed as limiting the scope of the appended claims.

According to a general aspect of the present disclosure, the analysis system is an automatic or automated analysis system. The term "automatic" is to be understood such that - if appropriate after an initialisation step, which for example may comprise providing the aqueous test liquid in a sample chamber and/or starting a software application - the analysis of components (e.g., contaminants or other substances dissolved or suspended in the liquid) in the aqueous test liquid is conducted without the necessity for a user to engage with the system. This includes any steps of measurement and analysis with the deep UV spectroscopy system as well as calibration steps, but likewise any steps of supplying and removing the aqueous test liquid and/or a cleaning liquid to and from the sample chamber and any steps of cleaning or rinsing the sample chamber. The analysis system is preferably provided in such a manner that, after installation, there is no need for a user to engage with any major parts of the system, apart from possibly adjusting software settings, such as for example determining freely selectable time intervals at which measurements should be carried out.

According to a general aspect of the present disclosure, the analysis system is preferably an inline analysis system. The term "inline" or "inline measurement" is to be understood such that the aqueous test liquid is provided in a flow-through system. The analysis system is typically configured to draw the aqueous test liquid from a large scale liquid flow or large scale liquid reservoir and release the aqueous test liquid after the analysis is concluded to an outlet. The analysis system is in fluid communication with the large scale liquid flow or the large scale liquid reservoir.

According to another general aspect of the present disclosure, the analysis system is preferably an on-site analysis system. The term "on-site" describes that the analysis of the aqueous test liquid is conducted in the close vicinity, for example less than 100 m or even less than 30 m, of the large scale liquid flow or the large scale liquid reservoir to be analysed. The analysis system is preferably not intended as an off-site laboratory for analysis of previously collected aqueous test liquids.

According to another general aspect of the present disclosure, the analysis system is a real-time analysis system. The term "real-time" describes that the measurement and the analysis of the aqueous test liquid are concluded rapidly, and the user is provided with information on the analysis results for the aqueous test liquid rapidly, for example within less than 10 minutes, and preferably less than 1 minute.

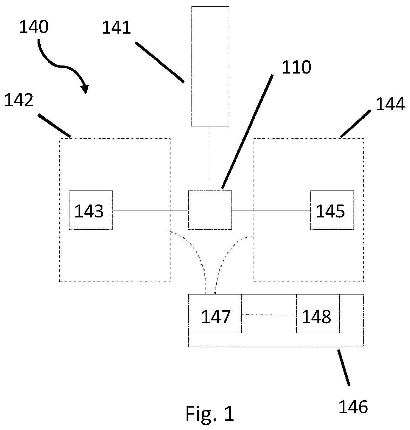

Figure 1 schematically illustrates an analysis system 100 for analysing of components in an aqueous test liquid, in particular a deep UV spectroscopy system 140, a sample chamber 110, and a processing system or controller 146, according to an embodiment of the present disclosure.

The sample chamber 110 is adapted for holding the aqueous test liquid and a cleaning liquid, and is typically in fluid communication with a liquid supply system (not shown in Figure 1). The sample chamber 110 includes at least one transparent wall portion. The transparent wall portion is transparent for the wavelength range of an excitation beam of the deep UV spectroscopy system 140. Typically, the transparent wall portion is transparent for at least a wavelength range of 190 nm to 600 nm. The sample chamber 110 may include a second or further transparent wall portion for allowing the excitation beam to exit the sample chamber 110 and/or for allowing radiation emitted by the aqueous test liquid to reach a measurement set-up, and in particular a detector, of the deep UV spectroscopy system 140. The second or further transparent wall portion may be transparent for at least a wavelength range of 190 nm to 300 nm, or even at least a wavelength range of 190 nm to 600 nm. The second or further transparent wall portion may be arranged in a wall portion of the sample chamber 110 which is opposite to the transparent wall portion, or arranged in a side wall of sample chamber 110.

The deep UV spectroscopy system 140 is provided for spectroscopic analysis of the aqueous test liquid, and optionally of the cleaning liquid, being held in the sample chamber 110. The deep UV spectroscopy system 140 includes a radiation source 141 for irradiating the aqueous test liquid in the sample chamber 110 by an excitation beam penetrating through the transparent wall portion. The radiation source 141 emits radiation in the deep UV spectral range. The term "deep UV" is to be understood as radiation with a wavelength between 190 nm and 270 nm. The radiation source 141 preferably emits radiation with a wavelength between 220 nm and 250 nm, and most preferably the wavelength is at least 230 nm and/or at most 250 nm. The radiation emitted by the radiation source 141 is narrow in its wavelength range, i.e. the Full width at half maximum (FWHM) may be substantially less than 1 nm. The radiation source 141 may be a laser source, wherein the laser source is preferably selected from the group consisting of a microchip laser unit, a solid state laser, a gas laser, or a hollow cathode ion laser.

The deep UV spectroscopy system 140 includes a first measurement set-up 142, and preferably a second measurement set-up 144, and optionally a third or further measurement set-ups.

In a preferred embodiment, as illustrated in Figure 1, the deep UV spectroscopy system 140 includes the first measurement set-up 142 for measuring a Raman spectrum, and a second measurement set-up 144 for measuring a photoluminescence spectrum.

According to an aspect, the first measurement set-up 144 is configured for measuring inelastically scattered radiation caused by the radiation source 141 and having been inelastically scattered by the aqueous test liquid. The first measurement set-up 144 includes a first detector 145. The first detector 145 may be any spectrometer, in particular the first detector 145 may be a Michelson spectrometer or a Fourier-transform spectrometer. By irradiating the aqueous test liquid with a wavelength in the deep-UV wavelength range, the intensity of Raman signals can be considerable due to the intensity of inelastically scattered radiation being nonlinearly dependent on the radiation frequency or photon energy. Thus, Raman spectroscopy carried out in the deep-UV can result in signal intensities which are orders of magnitude higher than signal intensities observed in "classic" Raman measurements, which commonly employ radiation sources with wavelengths in the visible to near-infrared wavelength range. Advantageously, the wavelength of the radiation source 141 can be chosen such that resonant Raman spectroscopy can be realised, which may result in even more considerable signal intensities. Therefore, the first measurement set-up 144 based on deep-UV Raman spectroscopy can be highly sensitive and therefore especially suitable for detecting components or contaminants with very low concentrations.

One of the drawbacks of carrying out Raman measurements at wavelengths shorter than the commonly employed visible to near-infrared wavelength range may be the occurrence of fluorescence radiation, wherein fluorescence signal intensities can be substantially greater than Raman signal intensities. However, empirically it was found that fluorescence signal intensities are very moderate or even negligible for most components when radiation sources 141 with a wavelength in the deep-UV are employed, preferably with wavelengths no larger than 250 nm. Suitable radiation sources 141 with wavelengths in the deep-UV wavelength range and in particular with wavelengths below 250 nm, which generate sufficient photon flux have only become technically feasible recently.

According to an aspect, the second measurement set-up 142 is suitable for measuring photoluminescence radiation emitted by the aqueous test liquid as a result of absorption of radiation by the aqueous test liquid, and in particular for detecting radiation with a wavelength between 260 nm and 600 nm. The second measurement set-up 142 may include a second detector 143. The second detector 143 may be any spectrometer, in particular the second detector 143 may be a Michelson spectrometer or a Fourier-transform spectrometer, and/or include an optical grating.

In some embodiments, the deep UV spectroscopy system 140 may include a third measurement set-up for measuring a further property. The further property may be selected from the group consisting of an opacity of a transparent wall portion of the sample chamber 110, a temperature of the test liquid, a conductivity of the test liquid, and a pH of the test liquid.

The processing system or controller 146 is adapted for controlling the analysis system 100. For example, the processing system may be adapted to control the liquid supply system, including supplying the aqueous test liquid and/or the cleaning liquid to the sample chamber 110. Further, the processing system 146 is adapted to control the deep UV spectroscopy system 140, including recording and processing any spectra of the aqueous test liquid being held in the sample chamber 110.

As a result of conducting a measurement with the first measurement set-up 144 a raw Raman spectrum of the test liquid being held in a sample chamber is generated. As a result of conducting a measurement with the second measurement set-up 142 a raw photoluminescence spectrum of the test liquid being held in a sample chamber is generated. The processing system 146 comprises a pre-processing unit 147 configured for receiving data generated by the second measurement set-up 142 and by the first measurement set-up 144. The pre-processing unit 147 may receive data due to an interconnection between each of the first and second measurement set-ups 142, 144 and the pre-processing unit 147 and may be based on a WiFi connection, a Bluetooth connection, a physical wire or on a printed circuit board. Furthermore, in embodiments comprising a third measurement set-up or even further measurement set-ups, the pre-processing unit 147 may also be configured for receiving data generated by the third measurement set-up and/or further measurement set-ups.

The present inventors discovered that the spectra recorded with aqueous test liquids irradiated by means of deep UV radiation, exhibit signals or peaks of water, which can be used to scale the respective spectra, and thereby enable the determination of concentrations of other components in the aqueous test liquids such as nitrates, without the need for utilising any additional spectroscopic methods or a separation of individual components of the aqueous test liquids.

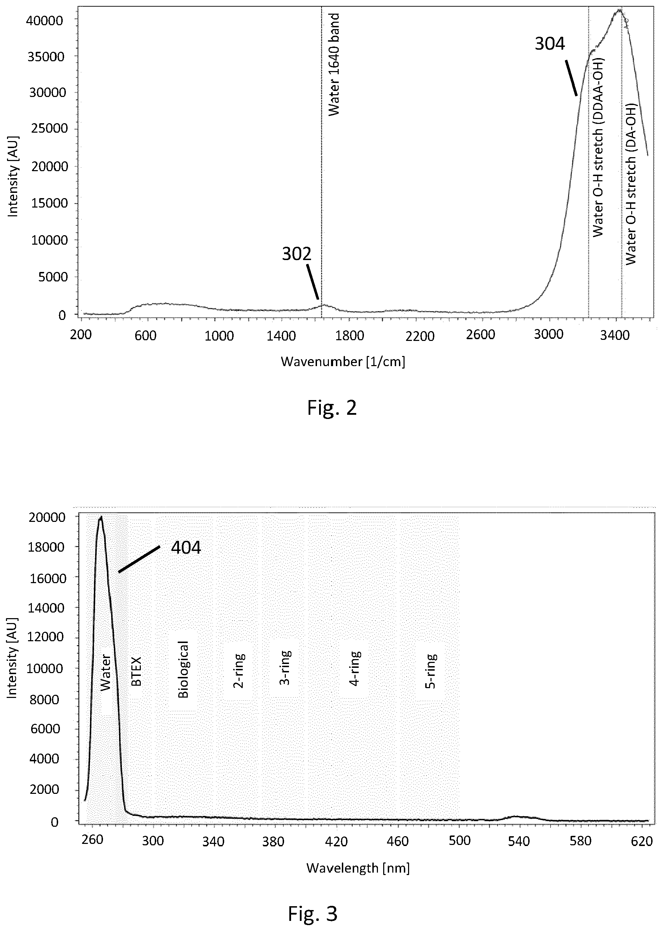

The raw Raman spectrum of the aqueous test liquid comprises the wavelength of a Raman peak of water. The Raman peak of water may also be referred to as the first peak of water. For example, Figure 2 displays a part of a Raman spectrum of a water sample, which is largely free of contaminants, recorded with the first measurement set-up 144 for measuring the Raman spectrum. The measured spectrum includes two peaks 302, 304. Peak 302 is visible at around 1640 cm⁻¹. Peak 304 extends from approximately 3000 cm-¹, peaks at approximately 3400 cm⁻¹, and includes two contributions each of which involve O-H stretching bands.

In a preferred embodiment, the Raman peak of water is a Stokes band of water. Typically, one of the peaks visible in the raw Raman spectrum is utilised by the processing system. Preferably, the Raman peak of water used for processing the Raman spectrum is the peak 302 at around 1640 cm⁻¹. The processing system is configured to extract the amplitude of the Raman peak of water from the raw Raman spectrum. Typically, the processing system 146 is configured to integrate the amplitude of the Raman peak of water. Further, the processing system is configured to determine from the extracted Raman peak of water a scaling factor. According to one aspect, the scaling factor may be the integrated amplitude value of the Raman peak of water. Alternatively, the scaling factor may also include further input parameters (as disclosed further below). The scaling factor may be constant or wavelength-dependent (i.e., the scaling factor being a wavelength-dependent function).

The processing system 146 is further configured to scale the raw Raman spectrum by the scaling factor. The scaling therefore includes an amplitude scaling of the raw Raman spectrum. The scaled Raman spectrum enables determining the concentrations of further components in the aqueous test liquids.

According to one aspect, the processing system 146 may be configured to identify further peaks in the scaled Raman spectrum. The processing system 146 may be adapted to identify or recognise which component or type of component the identified further peaks originate from. For example, the processing system 146 may compare the identified further peaks with known or expected peaks stored in the processing system. The processing system therefore is configured for qualitative analysis of the peaks identified in the scaled Raman spectrum. The identified further (first, second, third...) peaks may be associated with further (first, second, third) components in the aqueous test liquid.

The scaled Raman spectrum may be utilised to determine the concentrations of the further (first, second, third) components in the aqueous test liquid. The processing system typically includes a plurality of expected Raman spectra, or reference Raman spectra. The expected Raman spectra may be stored in the processing system. Preferably, the expected Raman spectra are derived from spectra previously recorded with the analysis system 100 for reference samples with calibration liquids. The calibration liquids typically essentially consist of water and one preselected further component, wherein the concentrations of each component are predefined. The calibration liquid may also essentially consist of water and a plurality of further components, wherein the concentrations of each component are predefined. Alternatively, the expected Raman spectra correspond to literature Raman spectra.

According to an aspect, the expected Raman spectra are derived from spectra previously recorded with the analysis system 100 by scaling the raw Raman spectra with the scaling factor. The scaling factor for the expected Raman spectra may be determined in the same manner as for the raw Raman spectrum of the aqueous test liquid, in particular based on the same Raman peak of water.

According to an aspect, the processing system 146 may be configured to determine the concentration of the first component by evaluating a deviation of a first peak associated with the first component between the scaled Raman spectrum of the aqueous test liquid and the expected Raman spectrum. Typically, the processing system 146 may be adapted to integrate the amplitude of the first peak of the scaled Raman spectrum of the aqueous test liquid and determine a deviation to the integrated amplitude of the first peak of the expected Raman spectra.

The aqueous test liquid may contain a plurality of components or contaminants. The processing system 146 may be configured to determine the concentrations of each of the plurality of components contained in the aqueous test liquid. Each signal observed in the raw or scaled Raman spectrum may be identified or associated with a specific component. The processing system may be adapted to determine the concentrations of each of the plurality of components from the scaled Raman spectrum of the aqueous test liquid, and in particular based on a plurality of expected Raman spectra, e.g. based on an expected Raman spectrum for each of the components identified in the raw or scaled Raman spectrum.

The processing system 146 may further include a machine learning algorithm 148 for determining, from the data generated by the second measurement set-up 142 and by the first measurement set-up 144, qualitative and quantitative information regarding the composition of the aqueous test liquid or the cleaning liquid. In particular, the machine learning algorithm 148 may be adapted to determine the concentrations of each of the one or more components in the aqueous test liquid and/or to identify peaks in the scaled Raman spectrum (and likewise in the scaled photoluminescence spectra disclosed further below) of the aqueous test liquid and identify which component or type of component the identified further peaks originate from.

The raw or scaled spectra may be analysed by the machine learning algorithm 148 employing a Convolutional Neural Network (CNN) or an artificial neural network (ANN) or principle component analysis (PCA) or any other statistical method suitable for recognising features and/or patterns. The machine learning algorithm 148 may be configured for background noise reduction and/or noise subtraction. The background noise reduction and/or noise subtraction may be carried out automatically and/or implicitly by the machine learning algorithm 148, preferably by the Convolutional Neural Network, based on a layer for feature recognition. This is contrary to typical analysis methods, which are based on modelling background noise by means of a mathematical function and subtracting this mathematical function from the data prior to processing the data further. The processing system 146 according to embodiments of the present disclosure may be configured for simultaneous background noise reduction and/or noise subtraction and one or more further data processing steps.

According to an embodiment, the machine learning algorithm 148 may employ a Convolutional Neural Network (CNN), wherein the Convolutional Neural Network (CNN) comprises the following layers: a deep network with input layer, a plurality of convolution layers for automatic feature recognition and a plurality of fully connected layers for classification with a final output layer. The machine learning algorithm can be improved and/or extended and/or replaced with a different algorithm or model, for example if new data becomes available or new anomalies are detected, on a regular base by a software update. Software updates may be automatic updates of the Convolutional Neural Network facilitated by a data update comprising loading a new configuration and/or definition, for example based on the open neural network exchange (ONNX) format or a custom-made format. A simple data update may advantageously circumvent the need for installing new software. Further details of features of the machine learning algorithm 148 may be found in Liu et al. (Analyst, 2017, 142, 4067-4074) and/or Zou et al. (Scientific Reports 7, 2017, Article number: 6186), which are both herein incorporated by reference in their entirety.

In test liquids, wherein multiple components contribute to a measured spectrum, a plurality of signals may overlap and thus encumber the interpretation of the measurement. Advantageously, by employing the machine learning algorithm 148 the feature recognition may result in reliable qualitative and quantitative information for each individual component in the aqueous test liquid.

The analysis system 100 according to embodiments of the present disclosure may be chemically selective and yield quantitative results regarding the concentration of components in the aqueous test liquid even when a plurality of different components are present in the test liquid, without the need for separation of individual components of the test sample prior to carrying out measurements with the analysis system 100, which may reduce the amount of time and effort required to analyse the aqueous test liquid. Advantageously, the analysis system 100 may substantially reduce the time required to carry out an analysis of the test sample and can be configured for on-site measurement of the test sample. Furthermore, due to short measurement times for the first and second measurement set-ups 142, 144 and rapid analysis of the data by the processing system 146, the analysis of the test sample may be carried out, and in particular displayed, in real-time. The analysis system 100 enables determining the concentrations of components in the aqueous test liquids in a reliable manner largely independent of external or environmental parameters influencing the evaluation. For example, a reliable evaluation of the concentrations of the components is ensured, even in case the output power of the radiation source varies or deposits on the transparent wall portion result in a decrease of the radiation impinging on the test liquid, as the evaluation is fully or largely based on the relative signal strengths observed in the measured (Raman, photoluminescence) spectra. Analysis systems for determining quantitative information on the components in the test liquid based on a combination of measured parameters, such as including a measured power of a laser source may become increasingly unreliable with time, for example due to an insufficient and progressively decreasing cleanliness of the sample cell affecting the evaluation.

The raw photoluminescence spectrum comprises the wavelength of a second peak of water. Although the second measurement set-ups may be adapted or designed for measuring photoluminescence spectra, the (first, second or third) measurement set-up may further be suitable for detecting other types of spectroscopic signals for which the measurement set-up is not primarily optimised for. For example, the measured spectrum ranging from 260 nm to 600 nm may include further signals, e.g. Raman signals, besides the photoluminescence signals. In other words, the second measurement set-up may be adapted to detect a first type (photoluminescence) of signal (for which the measurement set-up is primarily adapted) and a second type of signal.

According to one embodiment, the second measurement set-up is adapted to detect a first type (photoluminescence) of signal (for which the measurement set-up is primarily adapted) and a second type of signal, wherein the second peak of water of the aqueous test liquid corresponds to the second type of signal. The second peak of water may correspond to a Raman peak. Preferably, the second peak of water is a Stokes Raman band of water.

Figure 3 displays a spectrum of water recorded with the second measurement set-up 142 for measuring photoluminescence radiation. One intense peak 404 is visible between approximately 260-280 nm. This peak originates from a Raman signal, i.e. corresponds to peak 304 shown in Figure 2. Water as cleaning liquid shows very minor intensity fluorescence signals (i.e. the intensities of the first type of signal is minor), whereas the Raman signal at approximately 3400 cm⁻¹ (or at approximately 270 nm for the wavelength of the radiation source selected in this specific example) is intense. Figure 3 additionally sketches wavelength ranges in which contaminants, such as BTEX (benzene, toluene, and the three xylene isomers), biological components, or 2-5 ring aromatic compounds, are approximately observed in fluorescence spectra (especially when excited with a deep UV radiation source). By utilising the deep UV radiation source, a Raman peak of water is observed which is spectrally spaced apart from fluorescence signals of other components/contaminants. The Raman peak 404 is therefore particularly suitable as the second peak of water.

The processing system 146 may be configured to determine scaled photoluminescence spectra and determine a concentration of a first or further component from the scaled photoluminescence spectrum in a similar manner as disclosed above for the Raman spectra. The processing system 146 may be configured to extract the amplitude of the second peak of waterfrom the raw photoluminescence spectrum. Preferably, the second peak of water is a Stokes Raman band of water. Further, the processing system 146 may be adapted to determine, from the extracted second peak of water, a second scaling factor. According to one aspect, the scaling factor is the integrated amplitude value of the Raman peak of water. Alternatively, the scaling factor may also include further input parameters (as disclosed further below). The processing system 146 may be adapted to scale the raw photoluminescence spectrum by the second scaling factor.

The processing system 146 may be configured to identify or recognise further signals or peaks in the scaled photoluminescence spectrum. For example, the processing system 146, and in particular the machine learning algorithm, may compare the identified further peaks with known or expected peaks stored in the processing system. The processing system therefore is configured for qualitative analysis of the peaks identified in the raw or scaled photoluminescence spectrum. The identified further (first, second, third...) peaks may be associated with further (first, second, third) components in the aqueous test liquid.

The scaled photoluminescence spectrum may be utilised to determine the concentrations of the further (first, second, third) components in the aqueous test liquid. The processing system typically includes a plurality of expected Raman spectra, or reference Raman spectra. The expected photoluminescence spectra may be stored in the processing system. Preferably, the expected photoluminescence spectra are derived from raw spectra previously recorded with the analysis system 100 for reference samples with calibration liquids.

According to an aspect, the expected photoluminescence spectra are derived from spectra previously recorded with the analysis system 100 by scaling the raw photoluminescence spectrum with the second scaling factor.

According to an aspect, the processing system 146 may be configured to determine the concentration of the first component by evaluating a deviation of a first peak associated with the first component between the scaled photoluminescence spectrum of the aqueous test liquid and the expected photoluminescence spectra. Typically, the processing system 146 may be adapted to integrate the amplitude of the first peak of the scaled photoluminescence spectrum of the aqueous test liquid and determine a deviation to the integrated amplitude of the first peak of the expected photoluminescence spectra.

The aqueous test liquid may contain a plurality of components or contaminants. The processing system 146 may be configured to determine the concentrations of each of the plurality of components contained in the aqueous test liquid. Each signal observed in the raw or scaled photoluminescence spectrum may be identified or associated with a specific component. The processing system may be adapted to determine the concentrations of each of the plurality of components from the scaled photoluminescence spectrum of the aqueous test liquid, and in particular based on a plurality of expected photoluminescence spectra, e.g. based on an expected photoluminescence spectrum for each of the components identified in the raw or scaled photoluminescence spectrum.

According to an aspect, the first measurement set-up 144 and the second measurement set-up 142 have one or more components in common. For example, a detection unit of the first detector 145 and a detection unit of the second detector 143 may be the same. The first and second measurement set-ups 144, 142 may essentially differ by an optical grating, i.e. the first detector 145 may include a first grating and the second detector 143 may include a second grating. The deep UV spectroscopy system 140 may be configured for actuating the first and second grating, e.g. moving the first (second) grating in and out of a beam path for measuring a spectrum with the first (second) detector. Movement or actuation of components of the first and second measurement set-up 144, 142, and especially the first and second grating, can result in changes in the alignment of the first and second measurement set-up 144, 142 with respect to the sample chamber 110. For example, movement or actuation of the first and second grating can result in an unreliable calibration of the wavelength axis of the spectra derived from the first and second measurement set-ups 144, 142. Therefore, it may be expedient to verify the accuracy of the wavelength calibration, and, if appropriate, re-evaluate the wavelength calibration on a regular basis.

According to an aspect, the processing system is adapted for extracting a peak position of the Raman peak of water and/or the second peak of water. The peak position may, for example, be a centroid of the respective position on the detector, and may for example correspond to a specific pixel of the detector. The processing system may be adapted to determine the wavelengths, or in other words determinate a calibrated wavelength axis for, the Raman spectrum and/or photoluminescence spectrum based on an expected wavelength of the Raman peak of water and/or the second peak of water stored in the processing system. Advantageously, the analysis system 100 according to the present disclosure enables determining the wavelength calibration in every measurement of the aqueous test liquid without the need for separate calibration measurements.

Illustratively, the analysis system may be suitable for detecting one or more of the following exemplary contaminants in the aqueous test liquid: antibiotics, psychotropic drugs, corrosion inhibitors, antihypertensives, anti-epileptic drugs, antidepressants, analgesics, diuretics, beta blockers, X-ray contrast mediums, pesticides, biocides, medical chemicals and food chemicals such as: pesticides, hormones, anti-inflammatory medication (diclofenac and the like), Detergents, disinfectants, food processing water (greases and fats, insecticides, herbicides, petroleum/gasoline), PCBs, drugs, antidepressants, hormonal/contraceptive pills, chemical waste, fertilizers, nitrate, phosphate compositions, heavy metals, plastic/microplastic, and diamond-like carbon.

According to one aspect, the scaling factor and/or the second scaling factor is further based on a measurement of the third measurement set-up. The processing system 146, and in particular the machine learning algorithm 148, may determine the scaling factor and/or the second scaling factor in dependence of the measurement data of the third measurement set-up, for example for taking effects such as a pH dependence or a temperature effect on the measured Raman or photoluminescence spectra into account. According to one aspect, the processing system 146 may be configured to determine the wavelength of the radiation source 141, in particularfrom elastically scattered light measured by the first measurement set-up. The processing system 146, and in particular the machine learning algorithm 148 may determine the scaling factor and/or the second scaling factor in dependence of the wavelength of the radiation source. In cases in which the scaling factor and/or the second scaling factor is determined in dependence of measurement data of the third measurement set-up, the wavelength of the radiation source, and/or other parameters, the (second) scaling factor may be wavelength-dependent (i.e., the scaling factor being a wavelength-dependent function).

According to an embodiment, the analysis system 100 is a waste water analysis system 100 and the aqueous test liquid is waste water. The spectroscopic analysis can be carried out at any purification stage, thereby enabling monitoring the concentration of contaminants such as micropollutants, phosphor compositions or nitride. The analysis system 100 may be suitable for the analysis of water in water pipes, such as tap water pipes, sanitary pipes or heating pipes in houses or in the ground or water pipes for process heating, process cooling as well as commercial cooling systems and building cooling systems.

According to an embodiment, the analysis system 100 is a beverage and food water analysis system 100 and the aqueous test liquid is beverage water and/or food water. Similarly, as discussed above for the waste water industry, the analysis system 100 may be beneficial for analysing industrial production of food and/or beverages and/or drugs.

According to an embodiment of the present disclosure, a method for analysing of components in an aqueous test liquid is provided. The method may include an analysis system 100 according to any embodiment of the present disclosure.

## Claims

1. An analysis system (100) for analysing of components in an aqueous test liquid, comprising:
a deep UV spectroscopy system (140) having a first measurement set-up (144) for measuring a raw Raman spectrum of the test liquid being held in a sample chamber (110), the raw Raman spectrum comprising the wavelength of a Raman peak of water; and
a processing system (146) for processing the raw Raman spectrum, wherein the processing system (146) is configured to
- extract the amplitude of the Raman peak of water from the raw Raman spectrum;
- determine, from the extracted Raman peak of water, a scaling factor; and
- scale the raw Raman spectrum by the scaling factor.

2. The analysis system according to claim 1, wherein the spectroscopy system (140) has a second measurement set-up (142) for measuring a raw photoluminescence spectrum of the test liquid being held in a sample chamber (110), in particular wherein the second measurement (142) set-up is suitable for detecting radiation with a wavelength between 260 nm and 600 nm.

3. The analysis system according to claim 2, wherein the raw photoluminescence spectrum comprises the wavelength of a second peak of water; and wherein the processing system (146) is configured to
- extract the amplitude of the second peak of water from the raw photoluminescence spectrum;
- determine, from the extracted second peak of water, a second scaling factor; and
- scale the raw photoluminescence spectrum by the second scaling factor.

4. The analysis system according to claim 3, wherein second peak of water is a Raman peak.

5. The analysis system according to any one of the preceding claims, wherein the processing system (146) is further adapted to determine a concentration of a first component from the scaled Raman spectrum and/or from the scaled photoluminescence spectrum.

6. The analysis system according to claim 5, wherein the processing system (146) is adapted to determine the concentration of the first component based on an expected Raman spectrum and/or an expected photoluminescence spectrum stored in the processing system (146).

7. The analysis system according to claim 6, wherein the expected Raman spectrum and/or the expected photoluminescence spectrum is derived from a raw spectrum of a calibration liquid, wherein the calibration liquid essentially consists of water and the first component.

8. The analysis system according to any one of claims 5 to 7, wherein the processing system (146) is adapted to determine concentrations of each of a plurality of components from the scaled Raman spectrum and/or from the scaled photoluminescence spectrum, and in particular based on a plurality of expected Raman spectra and/or a plurality of expected photoluminescence spectra stored in the processing system (146).

9. The analysis system according to any one of the preceding claims, wherein the deep UV spectroscopy system (140) comprises a radiation source (141), wherein the radiation emitted by the radiation source (141) has a wavelength between 190 nm and 270 nm, preferably between 220 nm and 250 nm, and more preferably between 230 nm and 250 nm; and optionally wherein the radiation source (141) is a laser source.

10. The analysis system according to any one of the preceding claims, wherein the Raman peak of water and/or the second peak of water is a Stokes band of water.

11. The analysis system according to any one of the preceding claims, wherein the processing system (146) is adapted for extracting a peak position of the Raman peak of water and/or the second peak of water, and determining the wavelengths of the Raman spectrum and/or photoluminescence spectrum based on an expected wavelength of the Raman peak of water and/or the second peak of water stored in the processing system (146).

12. The analysis system according to any one of the preceding claims, wherein the spectroscopy system (140) comprises a third measurement set-up for measuring a further property selected from the group consisting of an opacity of a transparent wall portion of the sample chamber (110), a temperature of the test liquid, a conductivity of the test liquid, and a pH of the test liquid; optionally wherein the scaling factor and/or the second scaling factor is further based on a measurement of the third measurement set-up.

13. The analysis system according to any one of the preceding claims,
wherein the analysis system (100) is a waste water analysis system and wherein the test liquid is waste water; or
wherein the analysis system (100) is a beverage and/or food water analysis system and wherein the test liquid is beverage water and/or food water.

14. A method for analysing of components in an aqueous test liquid, comprising an analysis system (100) having a deep UV spectroscopy system including a first measurement set-up (144) for measuring a raw Raman spectrum of the test liquid being held in a sample chamber (110), the raw Raman spectrum comprising the wavelength of a Raman peak of water; and the method including processing the raw Raman spectrum, comprising:
- extracting the amplitude of the Raman peak of water from the raw Raman spectrum;
- determining, from the extracted Raman peak of water, a scaling factor; and
- scaling the raw Raman spectrum by the scaling factor.
